# EUROPEAN PATENT SPECIFICATION

(11) **EP 1 270 660 B1**
(45) Date of publication and mention of the grant of the patent: **22.12.2004**
(21) Application number: 02013499.5
(22) Date of filing: 17.06.2002
(51) Int. Cl.: C08K 5/3492, A61L 31/04, A61L 31/06

(54) **Medical materials sterilized by radiation and their ways in use**
Strahlungssterilisierte medizinische Materialien und deren Verwendung
Substances médicales stérilisées par radiation et leurs applications

(30) Priority: 21.06.2001 JP 2001228719
(43) Date of publication of application: 02.01.2003
(73) Proprietor: BMG INCORPORATED, Kyoto-shi, Kyoto-fu 601-8023 (JP)
(72) Inventor: Gen, Shokyu, Kyoto 611-0023 (JP)
(74) Representative: Albrecht, Thomas, Dr.

(56) References cited:
- WO-A-98/15199
- US-A- 5 998 551
- PATENT ABSTRACTS OF JAPAN vol. 2000, no. 20, 10 July 2001 (2001-07-10) & JP 2001 072851 A (TEIJIN CHEM LTD), 21 March 2001 (2001-03-21)

## Description

### BACKGROUND of THE INVENTION

### 1. Field of The Invention

Present invention relates medical polymer materials sterilized by radiation and their ways in use, particularly medical polymer materials showing small deterioration or decomposition by time passage by being exposed to radiation such as γ ray or a microwave.

### 2. Description of The Related Art

There are many kinds of raw materials employed as medical use, in which furthermore functional materials are expected to employ in medical field, notwithstanding metals, ceramics and polymers are pointed out as biomaterials. Properties of medical material includes sterilization in product stage as important factor, adding to desirable functions of medical material.

Sterilization is defined in Japan Pharmacopoeia as sterilizing or removing all microbes in materials. There are 12 methods of 5 groups in sterilized methods pointed out in Japan Pharmacopoeia, in which sterilized methods by high pressure vapor, ethylene oxide gas (EOG) and radiation are widely and generally employed as living body.

As sterilized condition of high pressure vapor is applied about 20 minutes at 121 °C of 1.0 kg/cm ²G saturated vapor pressure in relative simple auto-crave, it is possible to be employed in sterilization of such many kinds of medical materials of standing at high pressure and temperature. However sterilized methods are employed in almost no case of organic polymer materials, caused on deterioration or decomposition, while many kinds of metals and ceramics are possibly stood in high pressure and temperature.

On the other hand, speaking to EOG sterilized method, sterilization instruments are developed in order to improve effective, efficient and safety in sterilization, because EOG is known effective as a disinfectant. Moreover, sterilized method seems best in comparison with others in order to prevent EOG sterilization from deteriorating the raw materials, But EOG sterilized method recently come to avoid employing, because it is pointed out that EOG remains in medical materials after sterilization, and has bad effect on living body.

According to recent research, sterilized method employed in suture for operation, disposable surgical gown and a hypodermic syringe, comes to change from usual EOG to radiation sterilized methods, because of high toxicity of residual EOG and complicated packing.

Radiation sterilized method is took attention as other sterilized method, in which radial rays are such beam as particles including light, coming from radioactive disintegration and composing of α,β,γ rays, neutron and X ray. General beam for the sterilized method applying for medical polymer material, is known to employ γ ray coming from ⁶⁰Co and electron beam.

As γ and electron beams have no serious damage to general-purpose polymer materials such as polyethylene, polypropylene, polyethylene chloride, polystyrene and synthetic rubbers, those polymers are possible to sterilize by radiation and have widely applied in their case until quite recent.

However, it is pointed out that coloration, bad smells and oxidation for worse, happen to occur on passage of time, caused on generally sterilized irradiation amount of about 25 kGy, new sterilized methods such as plasma and ultra-violet methods are developing. The plasma and ultra-violet methods are partly applied as a simple and easy method, but has a very narrow application caused on permeability.

Radiation exposed effects on the polymer materials are distinguished by cross-linking and collapse of the polymer chains, independent on irradiated rate, but proportional to irradiated dose over wide range. Cross-linking is defined that radicals are generated by irradiation process, in which new connections are generated inner- and intra- polymer molecules, molecular weight of the polymer finally increases until infinity, and the cross-linked polymer is insoluble in all kinds of solvents. On the other hand, the collapse of the polymer is defined that polymers happen to deteriorate their molecular weight and mechanical properties, because the polymer chains are cut by irradiation.

The polymers are classified in cross-linked and collapsed type polymers dependent on radiation effect. Typical examples of cross-linked polymer are pointed out as polyethylene, polypropylene, nylon, polystyrene, polyester, natural rubber and silicon resin. On the other hand, collapsed type polymers include poly-methyl-methacrylate, Teflon, poly-isobutylene, collagen, cellulose and bio-decomposed polymers such as poly-lactic acid. However, even cross-linked type polymers happen inevitably to deteriorate mechanical properties similar to the collapsed type because the both polymer chains are generated radicals and cut by irradiation. Therefore, even polyethylene of easiest cross-linked polymer by radiation happens to deteriorate mechanical properties by time passage after irradiation. It is because radicals generated by irradiation do not extinct in short time, but remain long time as free radicals in raw material and cause to cut polymer chain by reacting with oxygen by passage time.

The polymers are heat-treated at higher temperature than 100°C in order to remove these free radicals. However, when the polymers are heat-treated several hours at higher temperature than 100°C in order to fabricate cup and plate for artificial hip and knee joints from ultra high molecular weight poly ethylene (UHMWPE), there is fear of deforming the product by remained strain.

Many polymer materials made of polypropylene sterilized by radiation are available for disposable medical materials such as suture for operation, hypodermic syringe, filter, surgical gown, and non-woven sheet. It is proposed the methods of compounding the propylene with hydrated rosin-methyl-ester (JOP Sho61-213243) or styrene resin (JOP Hei7-157922) in order to prevent deterioration and bad smell from sterilizing by radiation. However, it is not quite effective because the free radicals can not completely be removed from by the above additives.

Only several kinds of polymers including polypropylene and polyethylene are available for sterilizing by radiation among many cross-linked types of decomposable and bio-absorbable polymers. Reason why radiation sterilized method is not employed in even radiation cross-linked type polymers of nylon or poly-vinylidene fluorite, is because the polymers happen to partly cross-link each others and partly decompose at same time, generate oligomers and monomers, and cause toxicity. Furthermore, we cannot employ the method on the suture for surgical operation, in which there is request of mechanical properties maintaining in long time because of toxicity problem. Recently, notwithstanding poly methyl methacrylate and silicon resin are generally employed on contact lenses and ocular lenses according to excellent optical properties and biological adequate, EOG sterilization is inevitable in end product because radiation is hazard. It is problem that EOG remains in medical materials after sterilization. As eye is the most sensitive organ in comparison with other organs and tissues, it is afraid to give further bad effect on living body.

On the other hand, medical polymer materials for implantation recently include suture for operation made from decomposable and bio-absorbable polymer, artificial dure mater, bonding agent for broken bone and is expected to increase various uses. In spite of a fact that it passed more than 30 years since the suture for operation made from decomposable and bio-absorbable polymer was applied in clinic, EOG sterilized method is still employed. It is because the decomposable and bio-absorbable polymer is originally composed of chemical structure that is unstable in heat, light, radiation and moisture.

Sterilized methods by high pressure vapor of EOG and radiation are widely and generally employed as living body as mentioned above. What the targets of present invention are to solve the following problems of radiation sterilized method, seem to be brightest future.
(1) Prevention of medical material deterioration caused on irradiation.
( 2 ) Expansion of used way of radiation sterilized method until medical materials that are impossible to be employed in past.

Moreover, if it is possible to sterilize indecomposable and bio-absorbable polymer by radiation, we cannot guess that effect. Especially, if it is possible to apply radiation sterilized method for decomposable and bio absorbable polymer that are impossible for medical use in past, we cannot guess the usefulness in social and economical scopes.

### SUMMARY of THE INVENTION

Present invention provides medical material sterilized by radiation, comprising polymer composite using in living body, containing multifunctional triazine compounds at weight ratio range of 0.01 to 20 weight percent to the polymer.

The present invention shows the fabrication of polymer composite having good heat and radiation resistance, by preventing heat molding record and irradiation on sterilized processes from deteriorating molecular weight caused on heat and radiation decomposition of the polymer. It is possible that the polymer composite is applied for the medical field of decomposable and bio-absorbable polymers and even bio-nonabsorbent polymers such as suture of operation or bonding agent for broken bone as a result. Furthermore, it is possible that the polymer composite is applied for not only medical material but also food wrapping material of industrial use and including fibers, foaming goods and castings for agricultural use.

### DETAILED DESCRIPTION of THE INVENTION

The inventor of the present report has found after wholeheartedly researching above subject that all kinds of polymer material composites including triallyl isocyanurate compound that is one of multi-functional triazine group compounds, preventing the polymer from cutting, decomposing their chains and forming cross linkage between polymer chains, and preventing from generation of bad smells and deterioration of mechanical properties by irradiation, although the polymers seem to be impossible caused on producing stage after molding by irradiation. Medical material the polymers according to the present invention are selected from both bio-absorbable and undecomposed polymer, and bio-nonabsorbable polymer materials

. Medical materials of bio-absorbable and decomposed polymer according to the present invention, include natural polymers selecting of collagen, gelatin, chitin, chitosan, silk, cellulose, hyaluronic acid, microbe-produced polyester such as poly⁻ β -hydroxybutylate, albumin and dextrin, and bio-absorbable and decomposed synthetic polymers selecting of poly-glutamine, poly glycolic acid, poly-lactic acid, poly caprylic acid, poly dioxanon, tri-methylene carbonate, and their co-polymer such as glycolic acid- caprylic acid copolymer, lactic acid-caprylic acid copolymer, lactic acid- dioxanon copolymer, glycolic acid-tri-methylene carbonate copolymer, or poly peptide, poly phosphatase, poly butylene succinate and their blends.

On the other hand, as medical material according to present invention, polymer is selected from bio-nonabsorbable and undecomposed polymer materials consisting of poly propylene, poly ethylene, poly amide, poly ester, poly carbonate, poly fluorovinylidene, silicon, poly urethane, natural rubber, synthetic rubber, poly vinylchloride, poly acetal, poly styrene, styrene resin, poly acrylonitrile, poly tetra-fluoro-ethylene, ethylene-vinyl-alcohol copolymer, ethylene-vinylacetate copolymer, poly methylmethacrylate, poly hydroxyethyl methacrylate, and poly sulphone, or their blends.

Cross-linking agent of the polymer applied in present invention is the multi-functional triazine compound. Triallyl isocyanurate, triallyl metha-isocyanurate, triallyl(2,3 di bromo) iso cyanurate include in triazine compound and triallyl isocyanurate is preferred based on reactive monomer.

The triazine compound is added 0.01 to 20 weight percent, preferably 0.1 to 10 weight percent to the polymer before molding polymer material as fabricating method of medical material. It is preferred to fabricate uniform product quality caused on adding the triazine compound before molding.

Cross-linking between polymer chains is achieved by irradiation of 10 to 50 kGy at final producing stage after molding. It is not preferred because deterioration starts with higher than 50 kGy irradiation, and sterilization effect is shortage at lower than 10 kGy irradiation.

Furthermore, it is possible to blend the polymer and known heat stabilizer, antioxidant, ultraviolet absorbent, light stabilizer, colorant, antistat, lubricant, nucleating agent, fire retardant, filler within limit of not spoiling effect of the present invention. For example, it is effective to blend the polymer and antioxidant such as vitamin E or catechin.

Medical material according to the present invention is the ways in use, employing on suture for operation, artificial blood vessel, bonding agent for broken bone, dental material, wound protector, artificial skin, contact lenses, ocular lenses, artificial ligament, artificial valve, artificial joint scraping parts, mesh, medical non-woven, stint, clip, Hotchkiss, artificial dure mater, scafford in tissue regeneration, provender from adhesion, anatomosis splint, disposable hypodermic syringe, catheter, blood bag for infusion, tube, disposable surgical gown and glove, forming product, sheet and filter.

The medical materials of the present invention are possible to apply for not only medical uses, but also industrial uses including packing or wrapping. It is possible to improve mechanical properties and bio-decomposability based on irradiation for sterilization and cross-linking between polymer chains at final producing stage after molding in products of the industrial uses.

### EXAMPLE

We explain details of the present invention according to following examples, however, not restricting the scope of the invention by the explanation.

Test procedure of the tensile strength and elongation was performed according to deification of JIS L1017. We have examined the specimen by employing tensile-tester of Shimazu Auotogragh 100 type, in room maintained constant temperature of 25°C, humidity of 65 RH %, at spacemen length of 250 mm and test speed of 300 mm/min.

### Example 1

Polypropylene pellet composed of weight-average molecular weight of about 320,000, is added triallyl cyanurate of 2.0 weight percent, melt-spinning by simple type spinner and drawing 4 times by warm air circular type stretching machine. Produced polypropylene fiber was packed in Aluminum/Polyethylene laminated bag replaced by nitrogen gas, further irradiating electron ray of 25kGy.

Irradiated fiber showed properties of non-soluble but swelling in heated xylene, and gelation ratio of about 0.75, caused by having cross-linking structure. And tensile strengths and elongations at break of the fiber before and after irradiation, were 5.7 g/d and 31 %, and 6.1 g/d and 29 %, respectively.

### Comparative Example 1

Polypropylene pellet was melt-spun by simple type spinner, same to example 1, but without adding triallyl cyanurate, melt-spinning by simple type spinner and drawing 4 times by warm air circular type stretching machine. Produced polypropylene fiber was packed in Aluminum/Polyethylene laminated bag replaced by nitrogen gas, further irradiating electron ray of 25kGy.

Irradiated fiber showed properties of soluble in heated xylene, and gelation ratio of about 0 percent. And tensile strengths and elongations at break of the fiber after irradiation, were 3.2 g/d and 21 %.

### Example 2

Poly vinylidene fluorite pellet composed of weight-average molecular weight of about 680,000, is added triallyl cyanurate of 1.0 weight percent, melt-spinning by simple type spinner and drawing 5 times in polyethylene bath at 150°C. Produced poly vinylidene fluorite fiber was packed in Aluminum/Polyethylene laminated bag under decompressed pressure, further irradiating Co60 γ ray of 25kGy.

Irradiated fiber showed properties of non-soluble but swelling in dimethyl formamide, and gelation ratio of about 0.68, caused by having cross-linking structure. And tensile strengths and elongations at break of the fiber before and after irradiation, were 5.8 g/d and 27 %, and 6.2 g/d and 25%, respectively.

### Comparative Example 2

Poly vinylidene fluorite pellet was melt-spun by simple type spinner, same to example 2, but without adding triallyl cyanurate, melt-spinning by simple type spinner and drawing by warm air circular type stretching machine. Produced polypropylene fiber was packed in Aluminum/Polyethylene laminated bag under decompressed pressure, further irradiating Co60 γ ray of 25kGy.

Irradiated fiber showed properties of soluble in heated dimethyl formamide, and gelation ratio of several percents. And tensile strength and elongation at break of the fiber after γ ray irradiation, were 3.9 g/d and 20%.

### Example 3

Dried nylon 6 pellet composed of weight-average molecular weight of about 55,000, is added triallyl cyanurate of 0.8 weight percent, melt-spinning by simple type spinner and drawing 4 times by sandwiching between 2 heated rollers. Produced nylon 6 fiber was packed in Aluminum/Polyethylene laminated bag replaced by nitrogen gas, further sterilizing with respect to irradiating electron ray of 25kGy.

Irradiated fiber showed properties of non-soluble but swelling in heated m-cresol, and gelation ratio of about 0.66. And tensile strengths and elongations at break of the fiber before and after irradiation, were 6.3 g/d and 29 %, and 6.8 g/d and 27 %, respectively.

### Comparative Example 3

Nylon 6 pellet was melt-spun by simple type spinner, same to example 3, but without adding triallyl cyanurate, melt-spinning by simple type. Produced nylon 6 fiber was packed in Aluminum/Polyethylene laminated bag under decompressed pressure, further irradiating electron ray.

Irradiated fiber showed properties of soluble in heated m-cresol, and gelation ratio of 0 percent. And tensile strength and elongation at break of the fiber after electron ray irradiation, were 5.2 g/d and 23%.

### Example 4 and Comparative Example 4

Dried poly methylmethacrylate pellet composed of weight-average molecular weight of about 120,000, is added triallyl cyanurate of 2.0 weight percent, forming columns of 20mm diameter and 10 cm length by injection molder. After cutting the column to shape of ocular lens, produced poly methlmethacrylate column was packed in Aluminum/Polyethylene laminated bag replaced by nitrogen gas, further sterilizing with respect to irradiating Co60 γ ray of 25kGy.

Irradiated specimen showed properties of non-soluble but swelling in tetrahydrofuran, and gelation ratio of about 0.70. On the contrary, vacuum bag specimen (comparative example 4) that was formed and cut from injected column without adding triallyl cyanurate, and irradiated Co60 γ ray of 25kGy, was happened to turn yellow and bad smell caused on the decomposition, and was deteriorated to show the weight-average molecular weight of about 80,000.

### Example 5

Dried poly dioxaone (Hexa-fluoro iso propanol HFIP) solution composed of instrinsic viscosity of 2.5, is added triallyl cyanurate of 2.5 weight percent, getting monofilament fiber by spinning by simple type spinner, drawing and annealing. Produced monofilament fiber was packed in Aluminum/Polyethylene laminated bag replaced by nitrogen gas, further sterilizing with respect to irradiating electron ray of 25kGy.

Irradiated monofimament fiber showed properties of non-soluble but swelling in heated HFIP, and gelation ratio of about 0.77. And tensile strengths and elongations at break of the fiber before and after irradiation, were 7.7 g/d and 36 %, and 4.8 g/d and 35 %, respectively.

### Comparative Example 5

Poly poly dioxaone was melt-spun by simple type spinner, same to example 5, but without adding triallyl cyanurate, melt-spinning by simple type spinner, drawing and annealing. Produced monofilament fiber was packed in Aluminum/Polyethylene laminated bag under decompressed pressure, further irradiating electron ray.

Irradiated monofilament fiber showed properties of soluble in HFIP, and gelation ratio of 0 percent. And tensile strengths and elongations at break of the monofimament fiber after electron ray irradiation, were 3.1 g/d and 22%.

### Example 6

Dried poly L lactide (PLLA) pellet composed of weight-average molecular weight of about 340,000, is added triallyl cyanurate of 1.0 weight percent, forming rod columns of 10mm diameter and 10 cm length by injection molder. After solid-extruding the rod column at 140°C and as extruding ratio of 4, produced poly L lactide column was packed in Aluminum/Polyethylene laminated bag replaced by nitrogen gas, further sterilizing with respect to irradiating Co60 γ ray of 25kGy.

Irradiated column showed properties of non-soluble but swelling in methylen chrolide and gelation ratio of about 0.67. And bending strengths the column before and after irradiation, were 250 and 260MPa, respectively.

### Comparative Example 6

PLLA was formed by injection molder, same to example 6, and solid-extruded the rod column but without adding triallyl cyanurate. Produced PLLA was packed in Aluminum/Polyethylene laminated bag further irradiating Co60 γ ray of 25kGy.

Irradiated PLLA showed properties of soluble in methylene chrolide, and gelation ratio of 0 percents. And bending strength after γ ray irradiation, were deteriorated until 180MPa.

### Example 7

Ultra high molecule poly ethylene (UHMWPE) powder composed of weight-average molecular weight of about 4,500,000, is added triallyl cyanurate of 1.0 weight percent, forming plate of 10mm thickness by hot-press molder. Produced UHMWPE plate was packed in Aluminum/Polyethylene laminated bag replaced by nitrogen gas, further sterilizing with respect to irradiating Co60 γ ray of 25kGy.

Irradiated plate showed properties of non-soluble but swelling in hot tetralin solvent and gelation ratio of about 0.79. And abrasion resistance index evaluated by pin-on-flat tester before and after irradiation, were 0.9(×10⁻¹⁰ g/Nm) and 0.2(×10⁻¹⁰ g/Nm), respectively. Further, abrasion resistance index of 0.2 was not changed after oxidation test performed at 80°C in ambient air at one week.

### Comparative Example 7

Ultra high molecule poly ethylene (UHMWPE) powder composed of weight-average molecular weight of about 4,500,000, without adding triallyl cyanurate, was packed in Aluminum/Polyethylene laminated bag replaced by nitrogen gas, further sterilizing with respect to irradiating Co60 γ ray of 25kGy.

Irradiated plate showed properties of non-soluble but swelling in hot tetralin solvent and gelation ratio of about 0.58. And abrasion resistance index evaluated by similar method of example 7, is 0.6( × 10⁻¹⁰ g/Nm), however, abrasion resistance index of 14 was increased after oxidation test.

### Example 8

Dried poly glycolic acid pellet which showed η sp/c of 1.4 at 170 °C in solvent mixture of phenol(10) and 2.2.6 tri-chloro-phenol(7) in weight percent ratio, was added triallyl iso-cyanurate of 1.0 wt. percent, melt-spinning by simple type spinner, drawing, annealing and fabricating multifilament yarn. Produced multifilament yarn was packed in Aluminum/Polyethylene laminated bag replaced by nitrogen gas, further irradiating electron ray of 25kGy.

Irradiated multifilament yarn showed properties of non-soluble but swelling in solvent mixture at 170 °C and gelation ratio of about 0.48. And tensile strengths and elongations at break of the fiber before and after irradiation, were almost same values of 7.2 g/d and 22 %. Dried poly glycolic acid pellet added no triallyl iso-cyanurate, melt-spinning by simple type spinner, packing in Aluminum/Polyethylene laminated bag, showed the degradation of tensile strength and elongation at break as 3.8 g/d and 17 %.

### Example 9

Dried lactic acid and caproic acid copolymer pellet composed of weight-average molecular weight of about 420,000, was added triallyl iso-cyanurate of 1.0 wt. percent and vitamin E of 0.2 wt/ percent, melt-spinning by simple type spinner, drawing, annealing and fabricating monofilament yarn. Produced monofilament yarn was packed in Aluminum/Polyethylene laminated bag replaced by nitrogen gas, further irradiating electron ray of 25kGy.

Irradiated monofilament yarn showed properties of non-soluble but swelling in chloroform and gelation ratio of about 0.53. And tensile strengths and elongations at break of the fiber before and after irradiation, were almost same values of 5.7 g/d and 36 %.

Dried lactic acid and caproic acid copolymer adding no triallyl iso-cyanurate, melt-spinning by simple type spinner, packing in Aluminum/Polyethylene laminated bag, showed the degradation of tensile strength and elongation at break until 2.9 g/d and 21 %.

### Example 10

Dried poly L lactide (PLLA) pellet composed of weight average molecular weight of about 260,000, was added triallyl iso-cyanurate of 3.0 weight percent, forming film of 1.0mm thickness by hot-press molder, further irradiating electron ray of 50kGy in air atmosphere. Irradiated film showed properties of non-soluble but swelling in chloroform and gelation ratio of about 0.82.

The irradiated film showed good heat-resistant, because it was not softened even at higher temperature than 60 °C of glass transition point.

### Example 11

Dried lactic acid and caproic acid copolymer pellet composed of weight-average molecular weight of about 380,000 and component proportion of lactic acid (70 mol %) to caproic acid (30 mol %), was added triallyl iso-cyanurate of 3.0 wt. percent and vitamin E of 2.0 wt. percent, forming a film of 10 µm thickness by meting-film molder, further irradiating electron ray of 30kGy in air atmosphere. Irradiated film showed properties of non-soluble but swelling in chloroform and gelation ratio of about 0.79.

When frozen foods were packed by the irradiated film and defrosted by electric range for domestic use, the film did not change its shape and therefore, showed good heat-resistant.

## Claims

1. Medical material sterilized by radiation for use in the living body, containing a polymer and a multifunctional triazine compound as crosslinking agent for the polymer at a weight ratio in the range of 0.1 to 10 weight percent to the polymer; wherein
said polymer is a bio-absorbable and decomposable natural polymer or a bio-absorbable and decomposable synthetic polymer; and
irradiation of said polymer is made under nitrogen or under decompressed pressure, in an extent of 10 to 50 kGy.

2. Medical material according to claim 1, wherein said polymer is selected from the group of bio-absorbable and decomposable natural polymers consisting of collagen, gelatin, chitin, chitosan, silk, cellulose, hyaluronic acid, poly-β-hydroxybutylate, albumin and dextrin, or the group of bio-absorbable and decomposable synthetic polymers consisting of poly-glutamine, polyglycolic acid, polylactic acid, polycaprolactone, polydioxanone, tri-methylene carbonate, and their copolymer as well as poly phosphatase.

3. Medical material according to claim 1 or 2, wherein said extent of irradiation is about 25 kGy.

4. Medical material according to claim 1 or 2, further comprising Vitamin E or catechin.

## Patentansprüche

1. Durch Strahlung sterilisiertes medizinisches Material zur Verwendung im lebenden Körper, enthaltend ein Polymer und eine multifunktionelle Triazinverbindung als Vernetzungsmittel für das Polymer in einem Gewichtsverhältnis zu dem Polymer im Bereich von 0,1 bis 10 Gew.-%; wobei
das Polymer ein bioabsorbierbares und abbaubares natürliches Polymer oder ein bioabsorbierbares und abbaubares synthetisches Polymer ist; und
die Bestrahlung des Polymers unter Stickstoff oder vermindertem Druck und in einer Intensität von 10-50 kGy durchgeführt wird.

2. Medizinisches Material nach Anspruch 1, wobei das Polymer ausgewählt ist aus der Gruppe von bioabsorbierbaren und abbaubaren natürlichen Polymeren bestehend aus Collagen, Gelatin, Chitin, Chitosan, Seide, Cellulose, Hyaluronsäure, Poly-β-Hydroxybutylat, Albumin und Dextrin, oder der Gruppe von bioabsorbierbaren und abbaubaren synthetischen Polymeren bestehend aus Polyglutamin, Polyglykolsäure, Polymilchsäure, Polycaprolacton, Polydioxanon, Trimethylencarbonat und deren Copolymeren, sowie Polyphosphatase.

3. Medizinisches Material nach Anspruch 1 oder 2, wobei die Intensität der Bestrahlung etwa 25 kGy beträgt.

4. Medizinisches Material nach Anspruch 1 oder 2, welches des Weiteren Vitamin E oder Catechin umfasst.

## Revendications

1. Matériau médical stérilisé par un rayonnement, à utiliser dans le corps vivant, contenant un polymère et un composé de traizine multifonctionnel comme agen réticulant pour le polymère dans un rapport en masse de 0,1 à 10 % en masse par rapport au polymère; dans lequel
ledit polymère est un polymère naturel bio-absorbable et décomposable ou un polymère synthétique bio-absorbable et décomposable; et
l'irradiation dudit polymère s'effectue sous azote ou sous pression décompressée, à une dose de 10 à 50 kGy.

2. Matériau médical selon la revendication 1, dans lequel ledit polymère est choisi dans le groupe des polymères naturels bio-absorbables et décomposables constitué par le collagène, la gélatine, la chitine, le chitosane, la soie, la cellulose, l'acide hyaluronique, le poly-β-hydroxybutylate, l'albumine et la dectrine, ou dans le groupe des polymères synthétiques bio-absorbables et décomposables constitué par la polyglutamine, le poly(acide glycolique), le poly(acide lactique), la polycaprolactone, la polydioxanone, le carbonate de triméthylène et leurs copolymères, et la polyphosphatase.

3. Matériau médical selon la revendication 1 ou 2, dans lequel ladite dose d'irradiation est d'environ 25 kGy.

4. Matériau médical selon la revendication 1 ou 2, comprenant en outre de la vitamine E ou de la catéchine.
